(19) Europäisches Patentamt
European Patent Office
Office européen des brevets

(11) **EP 4 696 261 A1**

(12) **EUROPEAN PATENT APPLICATION**

(43) Date of publication:
**18.02.2026 Bulletin 2026/08**

(21) Application number: **25193160.6**

(22) Date of filing: **31.07.2025**

(51) International Patent Classification (IPC):
**A61B 18/02** (2006.01)

(52) Cooperative Patent Classification (CPC):
**A61B 18/02;** A61B 2018/00041; A61B 2018/00672;
A61B 2018/00678; A61B 2018/00702;
A61B 2018/00875; A61B 2018/00898;
A61B 2018/0293

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **12.08.2024 US 202418800551**

(71) Applicant: **Varian Medical Systems, Inc.
Palo Alto, CA 94304 (US)**

(72) Inventor: **ZHANG, Hongxuan
Austin, 78750 (US)**

(74) Representative: **Mathisen & Macara LLP
Charta House
30-38 Church Street
Staines-upon-Thames TW18 4EP (GB)**

(54) **CRYOABLATION APPARATUSES AND RELATED METHODS FOR PROLONGING LIFE OF HEATER IN CRYOPROBE**

(57) A cryoablation apparatus includes a cryoprobe (400) with a heater (406) and one or more sensors (408), and a cryo-controller with at least one processor and memory. The cryo-controller is configured to obtain cryoprobe operating information characterizing one or more operating characteristics of the heater (406), compare the cryoprobe operating information to a predetermined operating level of the heater to detect when a heater event occurs, and adjust a power signal supplied to the heater when the heater event is detected.

FIG. 4

**Description**

FIELD

The present disclosure relates to cryoablation apparatuses and related methods for prolonging life of heaters in cryoprobes.

BACKGROUND

This section provides background information related to the present disclosure which is not necessarily prior art.

Systems and methods for providing cryoablation treatments may include cryoablation probes that are introduced at or near target tissue in a patient. A cryoablation system may include an extremely cold cryogen (liquid, gas, or mixed phase) that may be passed through the probe that is in thermal contact with the target tissue. Heat from the tissue passes from the tissue, through the probe, and into the cryogen that removes heat from the targeted tissue. This removal of heat causes tissue to freeze, resulting in the destruction of the targeted tissue. It is desirable that the cryogen is of sufficiently low temperature to quickly and efficiently cause the targeted tissues to freeze.

It may also be desirable to keep the size of the needle of the cryoprobe to a small diameter or outer profile in order to be inserted accurately within a patient to target the target abnormal tissue while avoiding damage to healthy tissues. It may be difficult to maintain small outer diameters of the needle and still include desired components within the needle that may be desired to perform the cryoablation or related processes. There exists a need, therefore, for improved cryoablation apparatuses and related methods that overcome the shortcomings of existing cryoablation systems and methods. Such improved apparatuses and related methods may provide more robust systems and methods that can be used despite challenges that exist with maintaining sufficiently small diameters of cryoprobes.

SUMMARY

This section provides a general summary of the disclosure, and is not a comprehensive disclosure of its full scope or all of its features.

In various embodiments of the present disclosure, a cryoablation apparatus is provided as defined in the appended claims. The cryoablation apparatus may include a cryoprobe comprising a heater and one or more sensors and a cryo-controller with at least one processor and memory. The cryo-controller maybe configured to obtain cryoprobe operating information characterizing one or more operating characteristics of the heater, compare the cryoprobe operating information to a predetermined operating level of the heater to detect when a heater event occurs, and adjust a power signal supplied to the heater when the heater event is detected. The cryoablation apparatus may further comprise cryoablation console including the cryo-controller, a cryogen delivery apparatus and a cryogen source. The cryoablation apparatus may include a cryogen flow path that includes a cryogen supply line from the cryogen source to t he cryoprobe. The cryogen may flow back to the cryogen source via a cryogen return line or may be exhausted using a suitable exhaust valve. The cryoprobe suitably includes a needle, which may be a pointed cylindrical tool or other elongate member. The needle may have an outer diameter in a range of 1 mm to 4 mm. The cryoablation apparatus may further comprise a cryoprobe monitor. The cryoprobe monitor may be an electrical bus, a data acquisition unit, a computing device, a control board, circuit or other device that may receive cryoprobe operating information from the cryoprobe. The cryoprobe operating information may be, for example, temperature and/or impedance information that may be obtained using a sensor, thermocouple, temperature sensor, impedance sensor, or the like. The cryoprobe operating information may correspondence to a temperature and/or impedance obtained at one or more locations on or in the needle of the cryoprobe. The cryoprobe monitor may provide the cryoprobe operating information to the cryo-controller. The cryoprobe monitor may adjust, change, and/or modulate the power signal to the heater based on the cryoprobe operating information. In embodiments of the invention, the cryoprobe includes one or more measurement points. The measurement points may be coupled to the cryo-controller and may be used to measure one or more operating conditions of the cryoprobe. The measurement point may be a sensor, such as a thermocouple or electrical pads or contacts. The sensor may measure a resistance or impedance of the cryoprobe or of the tissue that may be located at or around the cryoprobe. The measurement points may be used to measure a resistance, impedance, temperature, or other characteristic of the cryoprobe.

In one aspect, the heater may be configured to raise a temperature of the cryoprobe to a temperature of 70 degrees Celsius or greater, such as 80 degrees Celsius or greater, such as 90 degrees Celsius or greater. The heater may be configured to raise a temperature of the cryoprobe to a temperature of up to 100 degrees Celsius. The temperature of the cryoprobe may be raised to any suitable cauterization temperature, which may be in a range of 70 degrees Celsius to 100 degrees Celsius. In other example, the cauterization temperature may be in the range of 80 degrees Celsius to 100 degrees Celsius. In other examples, the cauterization temperature may be in the range of 90 degrees Celsius to 100 degrees Celsius.

In another aspect, the one or more operating characteristics of the heater may include a resistance of the heater. Cryoprobes of the present disclosure may include resistive heaters that may be formed of coils or lengths of resistive wire. The wires used to form the heaters may have outer diameters less than 0.5 mm or less than 0.3 mm or less than 0.1 mm. The wires used to form the heaters may have outer diameters of at least 0.01mm, such as at least 0.05mm. The heater is suitably coupled to a suitable power source. The heater is suitably positioned at or near a tip of the needle. The heater may be used by the cryo-controller to measure one or more operating parameters or other operating information of the cryoprobe.

In another aspect, the heater may be positioned inside a needle or shell of the cryoprobe. A cryogen supply may also be located inside a needle or shell of the cryoprobe. The shell may be a tubular member that includes an outer wall that forms an inner cavity in which various elements of the cryoprobe may be enclosed. In an embodiment, the cryogen supply may be positioned centrally within the inner cavity of shell. The cryogen supply may be a suitable conduit or tube that can transport cryogen from a cryogen source to the tip of the cryoprobe. The cryogen may then flow away from the tip of the shell through the space between the outer surface of the cryogen supply and the inner surface of the shell. The heater may be positioned around the cryogen supply. The heater may be a resistive heater with a plurality of coils. The heater may be of a diameter such that it is located at or near the inner surface of the shell.

In another aspect, the heater may be positioned on and exterior surface of the shell. In another aspect the heater may be positioned embedded or fixed within the shell of a needle of the cryoprobe.

In another aspect, the predetermined operating level of the heater may be an upper resistance threshold and a lower resistance threshold.

In another aspect, the step of comparing the cryoprobe operating information to a predetermined operating level may include determining a cryoprobe operating index and comparing the cryoprobe operating index to one or more predetermined operating index distributions.

In another aspect, the one or more predetermined operating index distributions may include at least two predetermined operating index distributions.

In another aspect, the at least two predetermined operating index distributions may include a first predetermined operating index characterizing a baseline resistance of the heater and a second predetermined operating index characterizing a resistance of the heater in a short condition.

In another aspect, the heater event may include one of a short condition, an open circuit condition, or a failure condition.

In another aspect, the step of adjusting the power signal supplied to the heater may include decreasing the power signal to the heater when the heater event is determined to correspond to a short condition.

In another aspect, the cryo-controller may be further configured to continue operation of the heater when the cryoprobe operating information indicates that the heater returns to a baseline operating condition.

In another aspect, the cryo-controller may discontinue operation of the heater when the cryoprobe operating information indicates that a heater failure has occurred.

In another aspect, the cryo-controller may be further configured to send a notification when the cryo-controller detects that the heater event occurs. The term heater event may be used to describe the issues noted above that may include a short circuit event, an open circuit event, a heater damage event, a heater melting event, or the like.

In some embodiments of the present disclosure, a method of operating a heater of a cryoprobe is provided as defined in the appended claims. The method may include obtaining cryoprobe operating information from one or more sensors of the cryoprobe, comparing the cryoprobe operating information to a predetermined operating level of the heater to detect when a heater event occurs, and adjusting a power signal supplied to the heater when the heater event is detected. The cryo-controller may obtain the cryoprobe operating information from sensors or from the heater coil of the cryoprobe.

In one aspect, the step of comparing the cryoprobe operating information to a predetermined operating level may include determining a cryoprobe operating index and comparing the cryoprobe operating index to one or more predetermined operating index distributions. In some embodiments of the present disclosure, various parameters or indexes may be used as an alternative to and/or in combination with resistance values and/or power values of the heater of cryoprobes. Such parameters and indexes may correspond to resistances, temperatures, power usage, current usage, or other operating parameters of the cryoprobe and/or the heater. In various examples, a mean or average may be used. A standard deviation may be used. A signal variation may be used and/or a signal variability may be used. The parameters and/or indexes may be compared to known distributions or baseline distributions of such parameters and/or indexes to determine when heater events may occur. In some embodiments, an indexes or parameter may be calculated using real-time or current operating information for a characteristic of the cryoprobe such as resistance, temperature, power, or current. The real-time index may be compared to a baseline index that characterizes an anticipated operating condition of the heater.

In another aspect, the one or more predetermined operating index distributions may include at least two predetermined operating index distributions.

In another aspect, the at least two predetermined operating index distributions may include a first predetermined operating index characterizing a baseline resistance of the heater and a second predetermined operating index characterizing a resistance of the heater in a short condition.

In another aspect, the heater event may include one of a short condition, an open circuit condition, or a failure condition.

In another aspect, the step of adjusting the power signal supplied to the heater may include decreasing the power signal to the heater when the heater event is determined to correspond to a short condition.

Further areas of applicability will become apparent from the description provided herein. The description and specific examples in this summary are intended for purposes of illustration only and are not intended to limit the scope of the present disclosure.

DRAWINGS

The drawings described herein are for illustrative purposes only of selected embodiments and not all possible implementations, and are not intended to limit the scope of the present disclosure.

FIG. 1 is an illustration of an example cryoablation apparatus in accordance with some embodiments of the present disclosure.

FIG. 2 is an illustration of another example cryoablation apparatus in accordance with some embodiments of the present disclosure.

FIG. 3 is a side sectional illustration of an example cryoprobe in accordance with some embodiments of the present disclosure.

FIG. 4 is a side sectional illustration of another example cryoprobe in accordance with some embodiments of the present disclosure.

FIG. 5 is a side sectional illustration of another example cryoprobe in accordance with some embodiments of the present disclosure.

FIG. 6 is a side sectional illustration of another example cryoprobe in accordance with some embodiments of the present disclosure.

FIG. 7 is a plot of heater resistance data and heater power signal data during an example heater procedure illustrating aspects of the present disclosure.

FIG. 8 is a graph illustrating a signal pattern index that may be used to detect a heater event in accordance with some embodiments of the present disclosure.

FIG. 9 is a diagram illustrating an example machine learning model that may be used on some embodiments of the present disclosure.

FIG. 10 is a flow chart illustrating an example method of operating and monitoring a cryoprobe heater in accordance with some embodiments of the present disclosure.

FIG. 11 is a diagram illustrating an example computing device that may be used in some embodiments of the present disclosure.

Corresponding reference numerals indicate corresponding parts throughout the several views of the drawings.

DETAILED DESCRIPTION

Example embodiments will now be described more fully with reference to the accompanying drawings.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

When an element or layer is referred to as being "on," "engaged to," "connected to," or "coupled to" another element or layer, it may be directly on, engaged, connected or coupled to the other element or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

In some embodiments of the present disclosure, a cryoablation apparatus is provided that may be used to perform cryoablation treatments. The cryoablation apparatus may include a heater that may be used to perform repositioning of a needle and/or to perform cauterization procedures during a cryoablation treatment. The cryoablation apparatus may utilize the structures and methods described further in U.S Application No. 18/800,766 filed on 12 August 2024 to Varian Medical Systems, Inc entitled CRYOABLATION APPARATUSES AND RELATED METHODS FOR NEEDLE REPOSITIONING, the contents of which is hereby incorporated by reference herein in its entirety.

The heaters that may be used in the cryoprobes of the present disclosure may include one or more lengths of resistive wire that may heat up when a current is passed through the wire. The power that is supplied to the heater may be of sufficient levels to increase the temperature of the cryoprobe to a temperature of 80 degrees Celsius or greater and to hold this for a period of about 5 to about 20 seconds. In order to fit the heater into the cryoprobe or to mount the heater on the cryoprobe without increasing the overall size of the needle of the cryoprobe, the resistive wires of the heater may be wires with a small overall diameter. With manufacturing variation, the wires of the resistive heaters may be susceptible to failure or short. Some failures or shorts in the heater wires could cause a cryoablation treatment to be suspended. It is desirable, therefore, to detect occurrences during use of cryoprobe heater and take actions to prevent or minimize a likelihood of a short or failure that may cause suspension of the cryoablation treatment.

The cryoablation apparatuses and related methods of the present disclosure may monitor the use and performance of heaters in cryoprobes to detect when a short or failure condition is present. The cryoablation apparatuses and related methods may also determine operating conditions that may be used to prolong the life of the heater and allow a cryoablation treatment to continue without suspension. In some instances, the cryoablation apparatuses and methods may also determine that the cryoprobe is damaged or is in such a condition such that a cryoablation treatment should be suspended and the cryoprobe be discarded or repaired.

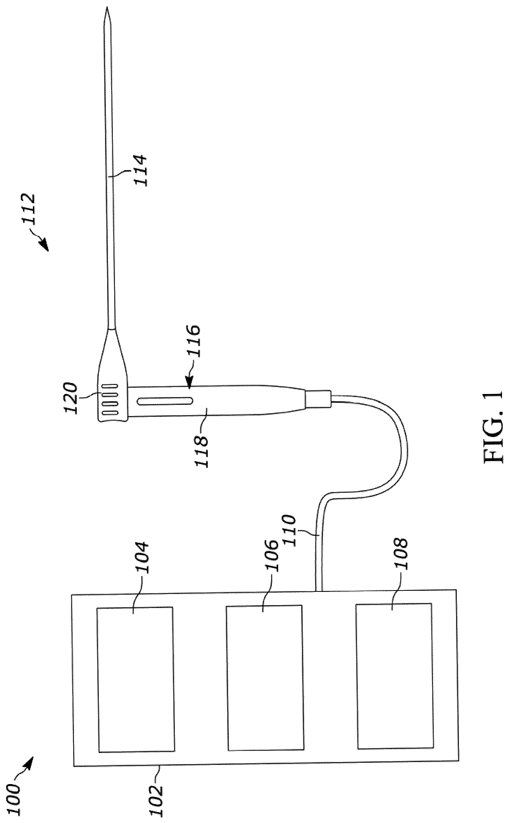

Referring now to FIG. 1, an example cryoablation apparatus 100 is shown. The cryoablation apparatus 100 may include a cryoablation console 102, and a cryoprobe 112. The cryoablation console 102 may include a cryo-controller 104, a cryogen delivery apparatus 106 and a cryogen source 108. The cryo-controller 104 may include a computing device or other controller that can be used to control delivery of a cryogen from the cryogen source 108 to the cryoprobe 112 using the cryogen delivery apparatus 106. The cryogen source 108 may be a suitable Dewar or other container that can be filled with a cryogen. The cryogen delivery apparatus 106 may include a pump, one or more valves, and other suitable fluid delivery devices to fluidly connect the cryogen source to the cryogen line 110 of the cryoprobe 112. Upon the initiation of a freezing cycle, the cryo-controller 104 may cause the cryogen to be moved through a cryogen flow path that includes a cryogen supply line from the cryogen source through the cryogen line 110 to the cryoprobe 112. The cryogen may then flow back to the cryogen source via a cryogen return line or may be exhausted from using a suitable exhaust valve.

The cryogen line 110 may be a flexible tube or other conduit that may include multiple lumens to allow the cryogen to flow in a supply direction to the cryoprobe 112 and separately in a return direction away from the cryoprobe 112. The cryogen line 110 may of sufficient length to allow the console 102 to be positioned near the patient in a treatment room and to allow the patient to be moved into and out of an imaging device. In some examples, the cryogen line may be at least about 3.65 meters (12 feet) in length. In other examples, the cryogen line 110 may have other lengths.

The cryogen line 110 fluidly couples the cryogen source 108 to the cryoprobe 112. The cryoprobe 112 may include a needle 114. The needle 114 may be a pointed cylindrical tool or other elongated member that is configured to be inserted into patient tissue and be positioned at or near the target tissue during treatment. The needle 114 may be configured as a pointed tool having an outer diameter in a range of about 1 mm to about 4 mm. The cryoprobe 112 may also include a handle 116. The handle 116 may be configured with a first (or proximate) portion 118 and a second (or distal) portion 120. The first portion 118 may be substantially aligned with the cryogen line 110 and the second portion 120 may offset at an angle relative to the first portion 118. The offset angle between the first portion 118 and the second portion 119 may be about 90 degrees to define a right angle handle. In other example, the first portion 118 and the second portion 120 may be offset at different angles.

In some examples, the handle 116 may include a vacuum chamber positioned at or near an outside surface of the handle 116. The vacuum chamber may insulate the exterior of the handle from the extremely low operating temperatures of the cryogen that moves through the handle to the cryoprobe 112. This may allow an operator to touch or otherwise manipulate the cryoprobe 112 during treatment.

The needle of the cryoprobe 112 may also include a heater positioned at or near the tip of the needle. The heater may be used to raise a temperature of the needle. The heater may be coupled to the console and suitable power source provided therein. The cryo-controller 104 may control a power signal delivered to the heater to raise a temperature of the needle. The temperature may be raised to a suitable cauterization temperature in a range of about 70 degrees Celsius to about 100 degrees Celsius. In other example, the cauterization temperature may be about 80 degrees Celsius or greater. In yet other examples, the cauterization temperature may be about 90 degrees Celsius to about 100 degrees Celsius.

While not shown, more than one cryoprobe 112 may be coupled to the console 102. Multiple cryoprobes 112 may be used during a single cryoablation treatment in combination. The console 102 may be configured to deliver cryogen to each of the multiple cryoprobes 112. The cryoprobes 112 may be similar to reach other or may be different to produce iceballs of different sizes and shapes so as to freeze and destroy the target tissue.

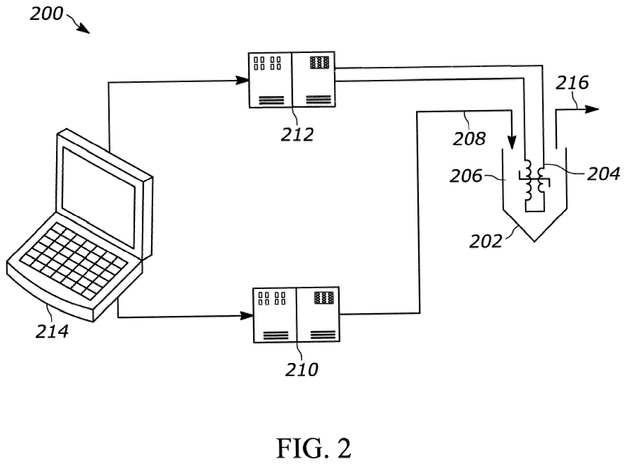

Referring now to FIG. 2, another example cryoablation apparatus 200 is shown. In this example, the cryoablation apparatus 200 may include a cryoprobe 202 operably coupled to a cryoablation control apparatus 214. The cryoablation control apparatus 214 may include a computing device such as a laptop, tablet, controller, programmable logic controller, smart device, or the like. The cryoablation control apparatus 214 may include a processor and memory. The memory may include executable instructions stored on the memory that may cause the cryoablation apparatus 200 to perform the functions and/or methods described herein.

The cryoablation apparatus 200 may include a cryogen delivery apparatus 210 and a cryoprobe monitor 212. The cryogen delivery apparatus 210 may include valves, pumps, and/or other cryogen delivery devices that may deliver a cryogen (e.g., liquid nitrogen) to the cryoprobe 202. The cryogen delivery apparatus 210 may be electrically coupled and/or controlled by the computing device of the cryoablation control apparatus 214. The cryogen delivery apparatus 210 may cause cryogen to flow to the probe in a supply line 208 and the be removed and/or vented from the cryoprobe 202 via a return or exhaust line 216.

The cryoprobe monitor 212 may also be electrically coupled to the computing device of the cryoablation control apparatus 214. The cryoprobe monitor may be an electrical bus, a data acquisition unit, a computing device, a control board, circuit or other device that may receive cryoprobe operating information from the cryoprobe 202. The cryoprobe operating information may be, for example, temperature and/or impedance information that may be obtained using a sensor, thermocouple, temperature sensor, impedance sensor, or the like. The cryoprobe operating information may correspondence to a temperature and/or impedance obtained at one or more locations on or in the needle of the cryoprobe 202. The cryoprobe monitor 212 may provide the cryoprobe operating information to the cryoablation control apparatus 214.

The cryoprobe 202 may be similar to the cryoprobe 112 previously described. In this example, the cryoprobe 202 may include a heater 204 positioned in a needle 206 of the cryoprobe 202. The heater 204 may be a resistive heater in some examples. The heater 204 may be coupled to the cryoprobe monitor 212. A temperature of the heater 204 and/or of the needle 206 may be obtained by the cryoprobe monitor 212. The cryoprobe monitor 212 may also provide a power signal to the heater 204. The cryoprobe monitor 212 may include a power supply or be coupled to a power supply to provide a desired power signal to the heater 204. The cryoprobe monitor 212 may adjust, change, and/or modulate the power signal to the heater 204 based on the cryoprobe operating information previously described. The cryoprobe monitor 212 and/or the cryoablation control apparatus 214 may adjust, change or modulate a suitable power signal to the heater 204 to cause the needle 206 of the cryoprobe 202 to achieve a desired cauterization or thaw temperature for a desired period of time.

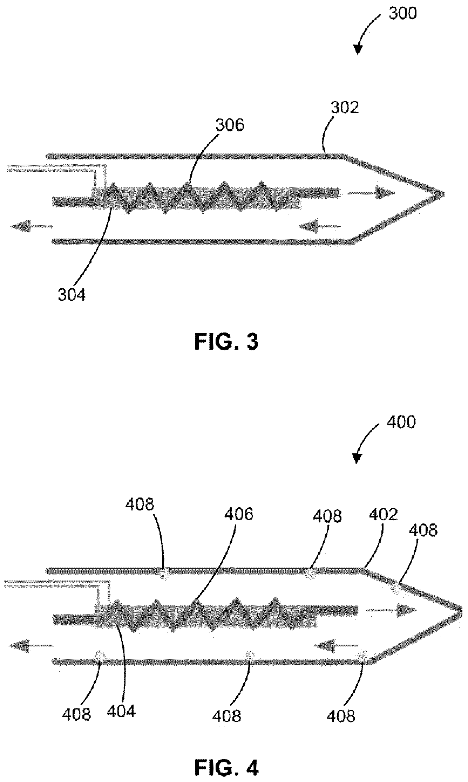

The cryoprobes that may be used in the cryoablation apparatuses and methods of the present disclosure may have various structures. The cryoprobes shown in FIGs. 3 to 6 illustrate some example cryoprobes. The cryoprobe 300 of FIG. 3 shows one example of a cryoprobe. The cryoprobe 300 may include a needle or shell 302, a cryogen supply 304 and a heater 306. The shell 302 may be a tubular member that includes an outer wall that forms an inner cavity in which various elements of the cryoprobe may be enclosed. In this example, the cryogen supply 304 may be positioned centrally within the inner cavity of shell 302. The cryogen supply 304 may be a suitable conduit or tube that can transport cryogen from a cryogen source to the tip of the cryoprobe 300. The cryogen may be moved toward the tip through the cryogen supply 304. The cryogen may then flow away from the tip of the shell 302 through the space between the outer surface of the cryogen supply 304 and the inner surface of the shell 302. With such flow, the cryogen may remove heat from the target tissue positioned around the cryoprobe to freeze and destroy the target tissue.

The heater 306 may be positioned around the cryogen supply 304. The heater 306 may be a resistive heater that may include a coil of resistive wire that heats when a power signal is passed through the heater 306. The heater 306 may be of sufficient resistance that the cryoprobe 300 may heat to a temperature suitable for cauterization. In some examples, such a temperature may be greater than or equal to 80 degrees Celsius.

The heater 306 may be coupled to the cryo-controller that may control a power signal supplied to the heater 306. The heater 306 may also be used by the cryo-controller to measure one or more operating parameters or other operating information of the cryoprobe 300. In some examples, the heater 306 may be used to measure a resistance, impedance, or temperature of the one or more locations in the cryoprobe 300. Such information may be used, as further explained below, to adjust or monitor the operation of the cryoprobe 300.

Referring now to FIG. 4, another cryoprobe 400 is shown. The cryoprobe 400 may be similar in many respects to the cryoprobe 300 previously described. The cryoprobe 400 may include a shell 402, a cryogen supply 404 and a heater 406. The shell 402, the cryogen supply 404, and the heater 406 may be similar to the similar elements described above with respect to cryoprobe 300. In this example, the cryoprobe 400 may additionally include one or more measurement points 408. The measurement points 408 may be coupled to the cryo-controller and may be used to measure one or more operating conditions of the cryoprobe 400. In various examples, the measurement points 408 may be a sensor, such as a thermocouple. In other examples, the measurement points 408 may be other sensors or electrical pads or contacts that may measure a resistance or impedance of the cryoprobe 400 or of the tissue that may be located at or around the cryoprobe 400. The measurement points 408 may be used to measure a resistance, impedance, temperature, or other characteristic of the cryoprobe 400.

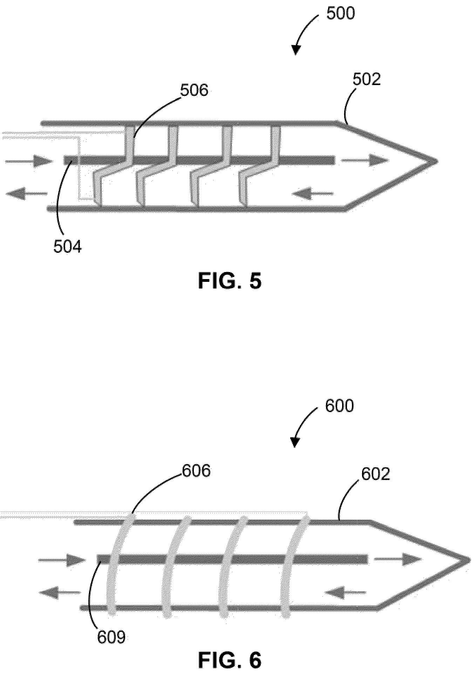

Referring now to FIG. 5, an example cryoprobe 500 is shown. The cryoprobe 500 may be similar to the cryoprobe 300 previously described in that the cryoprobe 500 includes a shell 502, a cryogen supply 504, and a heater 506. The shell 502 and the cryogen supply 504 may be similar to the shell 302 and the cryogen supply 304 previously described.

In this example, the heater 506 may be a resistive heater with a plurality of coils. The heater 506, in this example, may be positioned internal to the shell 502 and may be larger than the coil of heater 306. The coil of heater 506 may be of a diameter such that it is located at or near the inner surface of the shell 502. The heater 506 may be configured to allow measurement of cryoprobe operating information via the cryo-controller or other data processing or data acquisition unit.

Referring now to FIG. 6, another example cryoprobe 600 is shown. In this example, the cryoprobe 600 may include a shell 602 and a cryogen supply 604. The shell 602 and the cryogen supply 609 may be similar to the shell 302 and the cryogen supply 304 previously described. The heater 606, in this example, may be positioned on an exterior of the shell 602. The heater may be wrapped around the shell 602 or may be embedded or fixed in a wall of the shell 602 in some examples. The heater 606 may be a resistive heater formed of a coil of wire. The heater 606 may be used to collect cryoprobe operating information regarding one or more conditions of the cryoprobe 600 or the tissue.

While not shown, it should be appreciated that in other examples the cryoprobes 300, 500, 600 may include measurement points or other sensors such as that described in example cryoprobe 400. The measurement points or sensors may be used to collect temperatures, resistances, impedances, or other information regarding the operation of the cryoprobes.

As discussed above, the cryoprobes of the present disclosure may include resistive heaters that may be formed of coils or lengths of resistive wire. Such coils may heat when a power signal is supplied to the heater. The shells or needles of the cryoprobes of the present disclosure may have small outer diameters such as outer diameters in a range of about 1 mm to about 4 mm. With such sizing, the wires used to form the heaters of the present disclosure will have very small diameters. The wires may have outer diameters less than 0.5 mm or less than 0.3 mm or less than 0.1 mm. Manufacturing variation, manufacturing defects, and/or damage that may occur in the context of production, shipping, and operation of the cryoprobes may have a heightened impact on the operation of the heaters due to the extremely small size of the heaters required to be fit inside or on the cryoprobes.

Furthermore, in the context of cryoablation procedure, it is desirable to perform the procedure without interruption or delay due to the impact of the cryoablation on the patient. The cryoprobes and related methods of the present disclosure allow any issues with the cryoprobes and the heaters to be quickly identified. The heaters may be continued to be operated even in circumstances where issues may occur during use of the cryoprobe.

The heaters of the cryoprobes of the present disclosure may experience different types of issues during operation. In some instances a short circuit condition may occur. For example, due to a manufacturing defect, manufacturing variation, or damage, the wire of the heater may melt at the high temperatures used to perform cautery or other procedures during a cryoablation procedure. The wire of the heater may locally melt and bridge one or more coils of the heater. Such a short circuit condition may allow continued operation of the heater with modification. In other instances, the heater may melt or break and the heater could break or bridge to be in conductive contact with the shell of the needle. Such a condition would likely not allow continued operation of the cryoprobe.

In still other instances, the cryoprobe may experience an open condition. The heater of the cryoprobe could melt or break such that the continuity of the circuit is compromised. In such instances, operation of the cryoprobe likely would need to be discontinued. In other instances, the heater may experience local melting or deformation that may change a resistance of the heater but would allow for continued operation under modified operating conditions. In still other instances, other issues or operating changes of the heater may occur.

The cryoprobes and related methods of the present disclosure may identify the issues noted above. In the context of the present disclosure, the term heater event may be used to describe the issues noted above that may include a short circuit event, an open circuit event, a heater damage event, a heater melting event, or the like. The cryoprobes and related methods of the present disclosure may monitor the cryoprobe and heater operation to determine if and/or when a heater event has occurred and then take action to determine if operation of the cryoprobe may continue. In some instances, the cryoprobe may be continued to operate under modified operating conditions. This is an improvement over existing cryoprobes and methods that may not be able to determine if a heater event has occurred and will likely suspend or delay treatment rather being able to continue operation in some instances.

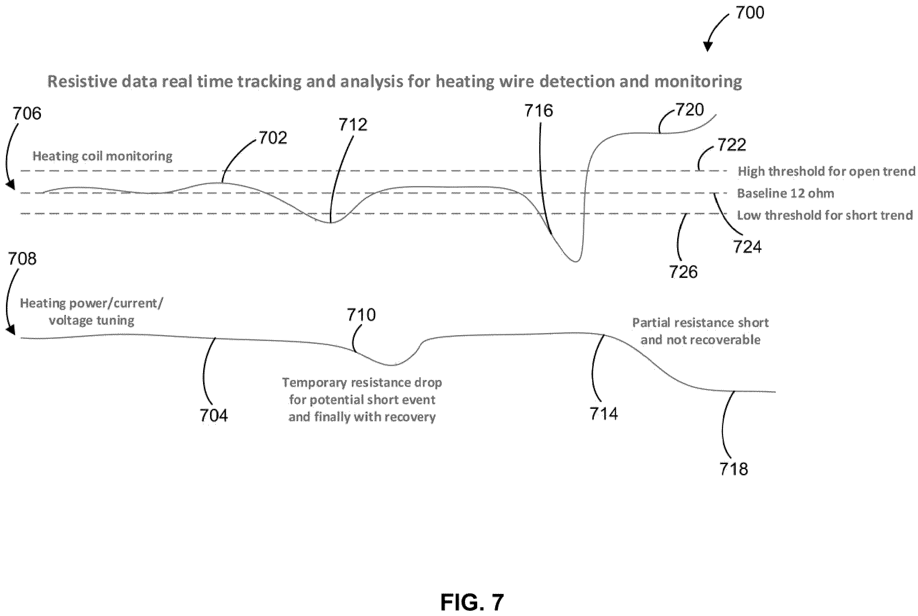

Referring now to FIG. 7, an example plot 700 of cryoprobe heater resistance data and cryoprobe heater power signal is illustrated. The plot 700 illustrates data that may be obtained from a cryoprobe during an example procedure in which a heater of a cryoprobe is energized. The heater may be energized to perform a cautery procedure during a cryoablation treatment. The heater resistance plot 706 may be obtained, for example, by the cryo-controller from the heater coil in a cryoprobe. The power signal plot 708 may be obtained, for example, by the cryo-controller from the heater or from the power supply coupled to the heater in the cryoprobe.

The plot 700 illustrates that during a cautery procedure (or other procedure in which the heater of the cryoprobe is energized) the operating conditions of the heater may be monitored to determine if and when a heater event occurs. In some examples, a baseline and one or more thresholds may be used to determine if a heater event occurs. In the example shown, the resistance of the heater may be monitored and compared to a baseline resistance 724, an upper resistance threshold 722, and a lower resistance threshold 726. The baseline resistance 724 in this example is a resistance of about 12 ohms. In other examples, the baseline resistance 724 may be other values depending on the construction and/or materials of the heater. The upper resistance threshold 722 may be a value at which the heater may operate without permanent damage to the heater. The upper resistance threshold 722 may be about 22 ohms in one example. In other examples other resistances may be used. The lower resistance threshold 726 may correspond to a value at which the heater may still be able to heat to desired temperature level and/or to a value that does not indicate a short has occurred that may indicate transmission of the power signal to the shell of the cryoprobe or to the tissue of the patient. In one example, the lower resistance threshold 726 may be about 2 to 5 ohms. In other examples, other lower resistance thresholds may be used.

As shown in FIG. 7, the heater resistance plot 706 illustrates an example cautery procedure in which a power signal is supplied to the heater. During the initial portions of the procedure indicated by portion 702 of the heater resistance plot 706 and portion 704 of the heater power signal plot 708, the heater may be operating in a normal manner and the resistance of the heater may be at or around the baseline resistance 724. At the portion 712 of the heater resistance plot 706 and at the portion 710 of the power signal plot 708, a heater event may be detected. The resistance of the heater may drop below the lower resistance threshold 726. Such a drop may indicate that a heater event such as a short circuit event has occurred. When such a heater event is detected, the cryo-controller may initiate a recovery action to determine if operation of the heater may continue. In the example shown and as indicated in the power signal plot 708, the power supplied to the heater may be decreased by the cryo-controller.

After dropping the power, the resistance is continued to be monitored. In the example shown, the resistance can be seen to increase and to recover such that the resistance returns to a level at or around the baseline resistance 724. In such circumstance, the operation of the heater may continue. The temporary drop in the power supplied to the heater allowed the heater to recover such that operation can continue.

At portion 716 of the heater resistance plot 706, a second heater even is detected. In this example, the resistance again drops to a level below the lower resistance threshold 726. Again, the cryo-controller may decrease a power delivered to the heater. As shown in this example event, the heater does not recover and initially continues to drop and then rapidly increases to a resistance level greater than the upper resistance threshold 722. Despite the cryo-controller continuing to drop the power to the heater, the resistance stays at a level greater than the upper resistance threshold 722. Such data or condition shown at portion 720 of resistance plot 706 and portion 718 of heater power signal plot 708 may indicate that an open circuit condition has occurred in the heater (e.g., permanent break or separation in the heater coil). When such a condition is detected, operation of the heater cannot continue since the heater will be unable to operate as intended. When such a heater event is detected, operation of the heater is suspended and the cryo-controller may alert or send a message to the user.

As can be appreciated, the plot 700 illustrates one example heater procedure in which two heater events are detected and the cryo-controller attempts to continue operation if the heater is able to recover. Other heater events may be detected in other circumstances and/or the cryo-controller may continue to operate the heater for multiple procedures if no heater events are detected.

In some embodiments of the present disclosure, various parameters or indexes may be used as an alternative to and/or in combination with resistance values and/or power values of the heater of cryoprobes. Such parameters and indexes may correspond to resistances, temperatures, power usage, current usage, or other operating parameters of the cryoprobe and/or the heater. In various examples, a mean or average may be used. A standard deviation may be used. A signal variation may be used and/or a signal variability may be used. Such parameters or statistical values may be characterized by the equations below for an operating parameter X of the heater and/or cryoprobe.

$$\text{Mean or averaging value (expectation); } mean(X) = \frac{1}{N}\sum_{i \in N} X(i);$$

$$\text{Standard deviation: } STD(X) = \frac{1}{N-1}\sum_{i \in N-1}(X(i) - mean(X))$$

$$\text{Signal Variation} = \frac{mean(X)}{STD(X)}$$

$$\text{Signal Variability} = \left|\frac{\max(X - mean(X))}{mean(X)}\right.$$

The parameters and/or indexes may be compared to known distributions or baseline distributions of such parameters and/or indexes to determine when heater events may occur. Various other indexes may also or alternatively be used to detect a heater event. In one example, an Index/Parameter Data Variation (IDV) may be used to detect a heater event. The IDV may be characterized using the equation below.

$$IDV = \frac{Mean(\text{Index value or ratio})}{STD(\text{Index value or ratio})}$$

In some embodiments, an indexes or parameter may be calculated using real-time or current operating information for a characteristic of the cryoprobe such as resistance, temperature, power, or current. The real-time index may be compared to a baseline index that characterizes an anticipated operating condition of the heater. The anticipated operating condition may indicate that the heater may continue to be operated to perform desired procedures such as thaw cycles, cautery procedures, or the like. The real-time index may also be compared to second, third, or other distributions that may correspond to an abnormal operating condition such as a heater event (e.g., short circuit event, open circuit event, damage event, etc.). In one example, the second distribution may correspond to a short circuit event in which the heater is able to recover after a lowering of power input and the third distribution may correspond to a short circuit event in which the heater is permanently damaged and is unable to continue operation. The real-time data distribution may be compared to all of the first distribution (normal operation), the second distribution (recoverable short circuit event), and the third distribution (unrecoverable short circuit event). By determining an overlap of the real-time distribution with each of the first distribution, the second distribution, and the third distribution, the cryo-controller may determine what action to take to continue operation (if possible) or to discontinue operation.

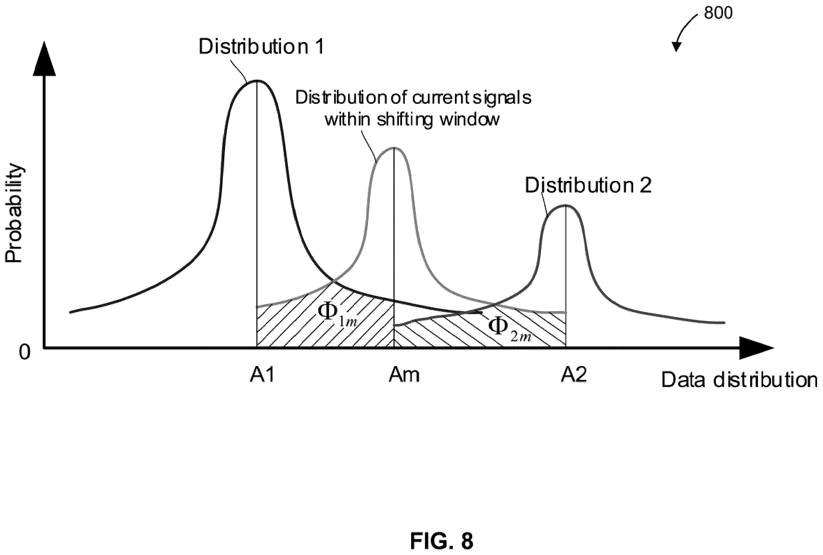

The amount of overlap between the distributions may be determined using a Signal Pattern function shown below in which m corresponds to the real-time data calculation and i corresponds to a pre-determined data distribution (e.g., the first distribution, the second distribution, or the third distribution). FIG. 8 illustrates a graphical depiction 800 of the Signal Pattern function. As shown, the Signal Pattern function below may determine the overlap which corresponds to an area of the real-time distribution that overlaps with one of the pre-determined data distributions for a heater event.

$$Signal\_pattern_{im} = \frac{\Phi_{im}}{\Phi_m} = \frac{\int_{mean_i - mean_m} Overlapping\_area(distribution\_i, distribution\_m)}{\int area(distribution\_m)}$$

In some examples, a machine learning model may be used to determine or detect when a heater event has occurred. The machine learning model may also be used to determine what action may be taken by the cryo-controller to continue operation of the heater and prolong use of the cryoprobe.

The term model or machine learning model as used in the present disclosure includes data models created using machine learning and/or artificial intelligence. Machine learning may involve training a mathematical model in a supervised or unsupervised setting. Machine learning models may be trained to learn relationships between various groups of data. The models may be based on a set of algorithms that are designed to model abstractions in data by using a number of processing layers. The processing layers may be made up of non-linear transformations. Machine learning models may include, for example, neural networks, convolutional neural networks and deep neural networks. Such neural networks may be made of up of levels of trainable filters, transformations, projections, hashing, and pooling. The models may be used in large-scale relationship-recognition tasks. The models can be created by using various open-source and proprietary machine learning tools and/or libraries known to those of ordinary skill in the art.

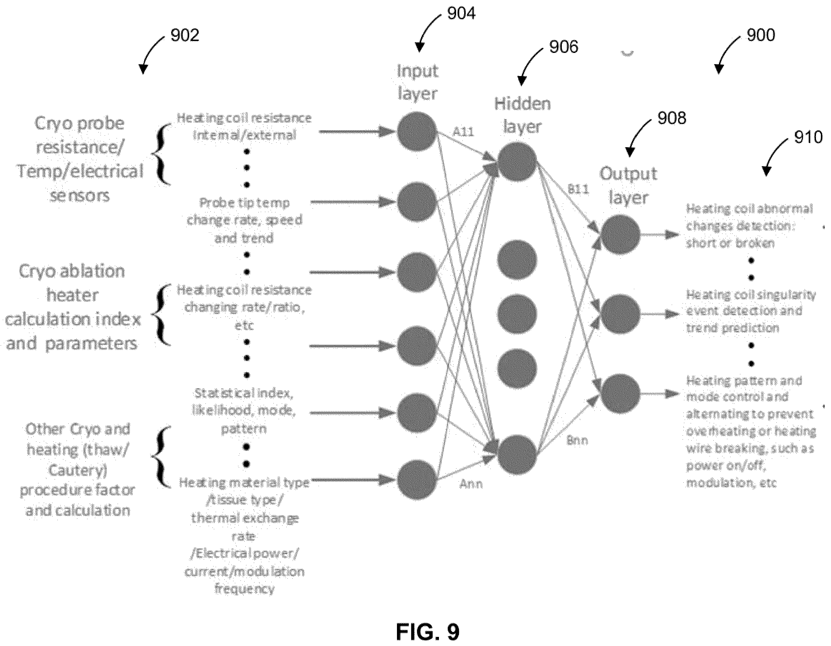

Referring now to FIG. 9, an example machine learning model 900 is shown. The machine learning model may be used by the cryo-controller and may be part of one or more cryoablation apparatuses of the present disclosure. The machine learning model 900 may use one more layers to process and/or identify complex, non-linear, or other relationships between the inputs and to recommend outputs that may allow the continued operation of the heater of the cryoprobe. In the example shown, the model 900 may utilize inputs 902 such as resistance, temperature, impedance, or other measurements made during a use of the heater. The inputs 902 may include rates of change of the measured parameters of the heater and/or the cryoprobe. The inputs 902 may also include parameters and indexes previously described. Still further inputs 902 may include a heater type, heater material, heater size, a tissue type, tissue location, patient information, power signal characteristics, current measurements, modulation characteristics and the like.

The inputs 902 may be provided to the model 900 and the multiple layers of the model such as the input layer 904, the hidden layer 906, and the output layer 908 may provide outputs 910. The outputs 910 may include information about whether a heater event has occurred, a type of heater event, and/or a trend prediction for operating parameters of the heater. The outputs 910 may also include a recommended heating pattern, power signal modulation, power signal characteristics, or others.

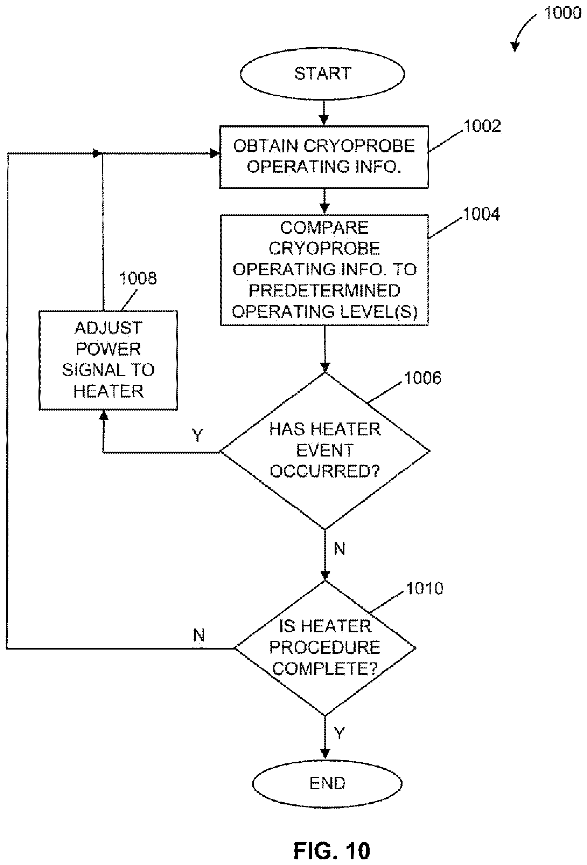

The present disclosure also contemplates various methods that may be performed by the heaters, cryoprobes, and cryoablation apparatuses of the present disclosure. In addition to the methods described above, the cryoablation apparatuses of the present disclosure may be operated to prolong a useful life of a cryoprobe. Referring now to FIG. 10, an example method 1000 is illustrated. The method may be performed by the cryoablation apparatus 100 or 200. The method 1000 is described as being performed by the cryoablation apparatus 100 but it should be appreciated that the method 1000 may be performed by other apparatuses.

The method 1000 may begin at step 1002. At step 1002, the cryo-controller 104 may obtain cryoprobe operating information. The cryo-controller 104 may obtain the cryoprobe operating information from sensors or from the heater coil of the cryoprobe 112. The cryoprobe operating information may characterize one or more operating characteristics of the cryoprobe 112 and/or the heater. The cryoprobe operating information may be a resistance of the heater, a temperature of the heater, and/or a power or current delivered to the heater in various examples.

At step 1004, the cryo-controller 104 may compare cryoprobe operating information to one or more predetermined operating levels. The cryo-controller 104 may compare real-time data or real-time data distributions to predetermined data thresholds, to predetermined or predicted data distributions. As previously described, the comparisons may include comparison of data distributions and/or compare calculated indexes or distributions to each other. In some examples, the cryoprobe operating information may be input to a trained machine learning model.

At step 1006, the cryo-controller may use the calculations and/or comparisons performed at step 1004 to determine if a heater event has occurred. The comparisons may allow the cryo-controller to determine if a short circuit event, an open circuit event, a damage event or other abnormal operation of the heater has occurred. If the cryo-controller 104 determines that a heater event has occurred, the method 1000 proceeds to step 1008. If the cryo-controller determines that a heater event has not occurred, the method proceeds to step 1010.

At step 1008, the cryo-controller may adjust a power signal provided to the heater of the cryoprobe. For example, the cryo-controller may adjust a current, or power of the power signal. The cryo-controller 104 may modulate the power signal. The cryo-controller 104, in some examples, may lower a power of the power signal if a short circuit event is detected. The cryo-controller 104 may lower the power of the power signal to determine if the heater may recover from the event and be able to continue operation. After step 1008, the method 1000 may return to step 1002 to continue to collect the cryoprobe operating information to determine if operation of the cryoprobe may continue. While not specifically shown, at step 1006, the cryo-controller may determine if the heater has recovered from an earlier detected heater event. The method 1000 may continue in the loop monitoring the cryoprobe operating information until it is determined that the heater has recovered or is operating in a normal range of operating parameters. If such is the case, the method 1000 may continue to step 1010 after recovery of the heater. If a heater event is continued to be detected at step 1006 after the power signal to the heater has been adjusted at step 1008, the cryo-controller may determine that operation of the cryoprobe should be discontinued. If such occurs, the cryo-controller 104 may alert or send a message to the user and shut-down operation of the heater.

At step 1010, the heater is operating in a normal range of operating conditions as a result of no heater events or after recovery of the heater. At step 1010, the cryo-controller 104 may determine whether the heater procedure is complete. Such heater may correspond to a cautery procedure, for example. The cryo-controller 104 may determine that the procedure is complete if a predetermined temperature has been reached and is maintained for predetermined period of time. In other examples, the cryo-controller 104 may determine a completion of the heater procedure using other measurements or data thresholds. If the cryo-controller determines that the heater procedure is complete, the method 1000 may end. If the cryo-controller 104 determines that the heater procedure is not complete, the method may return to step 1002. The steps of the method 1000 may be re-performed as previously described to continue to monitor a performance of the heater and to take action if a heater event is detected until such time that the heater procedure is determined to be complete at step 1010.

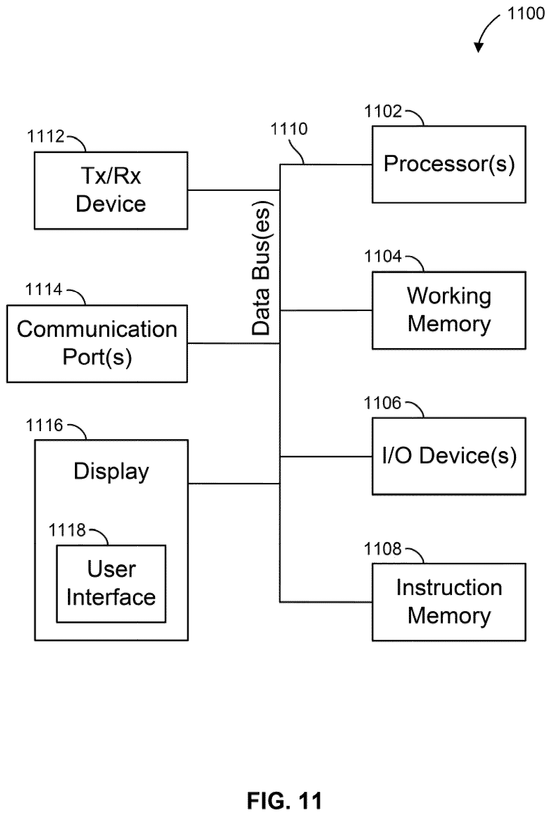

Referring now to FIG. 11, an example computing device 1100 is shown. The cryoablation apparatus 100, 200 may include one or more computing devices 1100. For example, the cryoablation computing device of console 102 and/or the cryo-controller 104 may have the elements shown in FIG. 1100. The cryoablation control apparatus 214 of the cryoablation apparatus 200 may include one or more computing devices 1100. The methods of the present disclosure, such as method 1000 may be performed, or steps of such methods may be performed, by a computing device 1100.

As shown, the computing device 1100 may include one or more processors 1102, working memory 1104, one or more input/output devices 1106, instruction memory 1108, a transceiver 1112, one or more communication ports 1114, and a display 1116, all operatively coupled to one or more data buses 1110. Data buses 1110 allow for communication among the various devices. Data buses 1110 can include wired, or wireless, communication channels.

Processors 1102 can include one or more distinct processors, each having one or more cores. Each of the distinct processors can have the same or different structure. Processors 1102 can include one or more central processing units (CPUs), one or more graphics processing units (GPUs), application specific integrated circuits (ASICs), digital signal processors (DSPs), and the like.

Processors 1102 can be configured to perform a certain function or operation by executing code, stored on instruction memory 1108, embodying the function or operation. For example, processors 1102 can be configured to perform one or more of any function, step, method, or operation disclosed herein.

Instruction memory 1108 can store instructions that can be accessed (e.g., read) and executed by processors 1102. For example, instruction memory 1108 can be a non-transitory, computer-readable storage medium such as a read-only memory (ROM), an electrically erasable programmable read-only memory (EEPROM), flash memory, a removable disk, CD-ROM, any non-volatile memory, or any other suitable memory.

Processors 1102 can store data to, and read data from, working memory 1104. For example, processors 1102 can store a working set of instructions to working memory 1104, such as instructions loaded from instruction memory 1108. Processors 1102 can also use working memory 1104 to store dynamic data created during the operation of cryoablation computing device of console 102. Working memory 1104 can be a random access memory (RAM) such as a static random access memory (SRAM) or dynamic random access memory (DRAM), or any other suitable memory.

Input-output devices 1106 can include any suitable device that allows for data input or output. For example, input-output devices 1106 can include one or more of a keyboard, a touchpad, a mouse, a stylus, a touchscreen, a physical button, a speaker, a microphone, or any other suitable input or output device.

Communication port(s) 1114 can include, for example, a serial port such as a universal asynchronous receiver/-transmitter (UART) connection, a Universal Serial Bus (USB) connection, or any other suitable communication port or connection. In some examples, communication port(s) 1114 allows for the programming of executable instructions in instruction memory 1108. In some examples, communication port(s) 1114 allow for the transfer (e.g., uploading or downloading) of data, such as cryoprobe operating information, resistance data, power data, temperature data, impedance data, and the like.

Display 1116 can display a user interface 1118. User interfaces 1118 can enable user interaction with the cryoablation computing device of console 102. For example, user interface 1118 can be a user interface that allows an operator to interact, communicate, control and/or modify different messages, settings, or features that may be presented or otherwise displayed to a user. The user interface 1118 can include a slider bar, dialogue box, or other input field that allows the user to control, communicate or modify a setting, limitation or input that is used in a cryoablation treatment. In addition, the user interface 1118 can include one or more input fields or controls that allow a user to modify or control optional features or customizable aspects of the cryoablation computing device of console 102 and/or the operating parameters of the cryoablation apparatus 100. In some examples, a user can interact with user interface 1118 by engaging input-output devices 1106. In some examples, display 1116 can be a touchscreen, where user interface 1118 is displayed on the touchscreen. In other examples, display 1116 can be a computer display that can be interacted with using a mouse or keyboard. The user interface is an example of the user interface 1118.

Transceiver 1112 allows for communication with a network. In some examples, transceiver 1112 is selected based on the type of communication network cryoablation computing device of console 102 will be operating in. Processor(s) 1102 is operable to receive data from, or send data to, a network, such as wired or wireless network that couples the elements of the cryoablation apparatus 100.

The following is a list of non-limiting illustrative embodiments disclosed herein:

Illustrative embodiment 1: A cryoablation apparatus comprising: a cryoprobe comprising a heater and one or more sensors; and a cryo-controller comprising at least one processor and memory, the cryo-controller configured to: obtain cryoprobe operating information characterizing one or more operating characteristics of the heater; compare the cryoprobe operating information to a predetermined operating level of the heater to detect when a heater event occurs; and adjust a power signal supplied to the heater when the heater event is detected.

Illustrative embodiment 2. The cryoablation apparatus of illustrative embodiment 1, wherein the heater is configured to raise a temperature of the cryoprobe to a temperature of 80 degrees Celsius or greater.

Illustrative embodiment 3. The cryoablation apparatus of any of illustrative embodiments 1 or 2, wherein the one or more operating characteristics of the heater comprises a resistance of the heater.

Illustrative embodiment 4. The cryoablation apparatus of any of illustrative embodiments 1 to 3, wherein the heater is positioned inside a needle of the cryoprobe.

Illustrative embodiment 5. The cryoablation apparatus of any of illustrative embodiments 1 to 4, wherein the heater is positioned on or in the shell of a needle of the cryoprobe.

Illustrative embodiment 6. The cryoablation apparatus of any of illustrative embodiments 1 to 5, wherein the predetermined operating level of the heater comprises an upper resistance threshold and a lower resistance threshold.

Illustrative embodiment 7. The cryoablation apparatus of any of illustrative embodiments 1 to 6, wherein the step of comparing the cryoprobe operating information to a predetermined operating level comprises determining a cryoprobe operating index and comparing the cryoprobe operating index to one or more predetermined operating index distributions.

Illustrative embodiment 8. The cryoablation apparatus of illustrative embodiment 7, wherein the one or more predetermined operating index distributions comprises at least two predetermined operating index distributions.

Illustrative embodiment 9. The cryoablation apparatus of illustrative embodiment 8, wherein the at least two predetermined operating index distributions comprises a first predetermined operating index characterizing a baseline resistance of the heater and a second predetermined operating index characterizing a resistance of the heater in a short condition.

Illustrative embodiment 10. The cryoablation apparatus of any of illustrative embodiments 1 to 9, wherein the heater event comprises one of a short condition, an open circuit condition, or a failure condition.

Illustrative embodiment 11. The cryoablation apparatus of any of illustrative embodiments 1 to 10, wherein the step of adjusting the power signal supplied to the heater comprises decreasing the power signal to the heater when the heater event is determined to correspond to a short condition.

Illustrative embodiment 12. The cryoablation apparatus of illustrative embodiment 11, wherein the cryo-controller is further configured to continue operation of the heater when the cryoprobe operating information indicates that the heater returns to a baseline operating condition.

Illustrative embodiment 13. The cryoablation apparatus of any of illustrative embodiments 11 or 12, wherein the cryo-controller discontinues operation of the heater when the cryoprobe operating information indicates that a heater failure has occurred.

Illustrative embodiment 14. The cryoablation apparatus of any of illustrative embodiments 1 to 13, wherein the cryo-controller is further configured to send a notification when the cryo-controller detects that the heater event occurs.

Illustrative embodiment 15. A method of operating a heater of a cryoprobe comprising: obtaining cryoprobe operating information from one or more sensors of the cryoprobe; comparing the cryoprobe operating information to a predetermined operating level of the heater to detect when a heater event occurs; and adjusting a power signal supplied to the heater when the heater event is detected.

Illustrative embodiment 16. The method of illustrative embodiment 15, wherein the step of comparing the cryoprobe operating information to a predetermined operating level comprises determining a cryoprobe operating index and comparing the cryoprobe operating index to one or more predetermined operating index distributions.

Illustrative embodiment 17. The method of illustrative embodiment 16, wherein the one or more predetermined operating index distributions comprises at least two predetermined operating index distributions.

Illustrative embodiment 18. The method of illustrative embodiment 17, wherein the at least two predetermined operating index distributions comprises a first predetermined operating index characterizing a baseline resistance of the heater and a second predetermined operating index characterizing a resistance of the heater in a short condition.

Illustrative embodiment 19. The method of any of illustrative embodiments 15 to 18, wherein the heater event comprises one of a short condition, an open circuit condition, or a failure condition.

Illustrative embodiment 20. The method of any of illustrative embodiments 15 to 19, wherein the step of adjusting the power signal supplied to the heater comprises decreasing the power signal to the heater when the heater event is determined to correspond to a short condition.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

**Claims**

1. A cryoablation apparatus (100, 200) comprising:

   a cryoprobe (112, 202, 300, 400, 500, 600) comprising a heater (204, 306, 406, 506, 606) and one or more sensors (408); and
   a cryo-controller (104, 214) comprising at least one processor and memory, the cryo-controller configured to:

      obtain cryoprobe operating information (1002) characterizing one or more operating characteristics of the heater (204, 306, 406, 506, 606);
      compare the cryoprobe operating information (1004) to a predetermined operating level of the heater (204, 306, 406, 506, 606) to detect when a heater event occurs (1006); and
      adjust a power signal (1008) supplied to the heater (204, 306, 406, 506, 606) when the heater event is detected.

2. The cryoablation apparatus of claim 1, wherein the heater is configured to raise a temperature of the cryoprobe (112, 202, 300, 40, 500, 600) to a temperature of 70 degrees Celsius or greater.

3. The cryoablation apparatus of claim 1 or 2, wherein the one or more operating characteristics of the heater (204, 306, 406, 506, 606) comprises a resistance of the heater.

4. The cryoablation apparatus of claim 1, 2 or 3, wherein the heater (204, 306, 406, 506) is positioned inside a needle (114, 206, 302, 402, 502) of the cryoprobe (112, 202, 300, 400, 500).

5. The cryoablation apparatus of claim 1, 2 or 3, wherein the heater (606) is positioned on or in the shell of a needle (602) of the cryoprobe (600).

6. The cryoablation apparatus of any one of the preceding claims, wherein the predetermined operating level of the heater (204, 306, 406, 506, 606) comprises an upper resistance threshold (722) and a lower resistance threshold (726).

7. The cryoablation apparatus of any one of the preceding claims, wherein the step of comparing the cryoprobe operating information to a predetermined operating level comprises determining a cryoprobe operating index and comparing the cryoprobe operating index to one or more predetermined operating index distributions.

8. The cryoablation apparatus of claim 7, wherein the one or more predetermined operating index distributions comprises at least two predetermined operating index distributions.

9. The cryoablation apparatus of claim 7 or 8, wherein the at least two predetermined operating index distributions comprises a first predetermined operating index characterizing a baseline resistance of the heater (724) and a second predetermined operating index characterizing a resistance of the heater in a short condition.

10. The cryoablation apparatus of any one of the preceding claims, wherein the heater event comprises one of a short condition, an open circuit condition, or a failure condition.

11. The cryoablation apparatus of any one of the preceding claims, wherein the step of adjusting the power signal supplied to the heater comprises decreasing the power signal to the heater when the heater event is determined to correspond to a short condition.

12. The cryoablation apparatus of any one of the preceding claims, wherein the cryo-controller (104, 214) is further configured to continue operation of the heater when the cryoprobe operating information indicates that the heater returns to a baseline operating condition (724).

13. The cryoablation apparatus of any one of the preceding claims, wherein the cryo-controller (104, 214) discontinues operation of the heater when the cryoprobe operating information indicates that a heater failure has occurred.

14. The cryoablation apparatus of any one of the preceding claims, wherein the cryo-controller (104, 214) is further configured to send a notification when the cryo-controller detects that the heater event occurs (712, 716, 720).

15. A method of operating a heater (204, 306, 406, 506, 606) of a cryoprobe (112, 202, 300, 40, 500, 600) comprising:

obtaining cryoprobe operating information (1002) from one or more sensors (408) of the cryoprobe;
comparing the cryoprobe operating information to a predetermined operating level (1004) of the heater to detect when a heater event occurs (712, 716, 720); and
adjusting a power signal supplied to the heater when the heater event is detected (1008), wherein, optionally, the step of comparing the cryoprobe operating information to a predetermined operating level comprises determining a cryoprobe operating index and comparing the cryoprobe operating index to one or more predetermined operating index distributions wherein, optionally, the one or more predetermined operating index distributions comprises at least two predetermined operating index distributions wherein, optionally, the at least two predetermined operating index distributions comprises a first predetermined operating index characterizing a baseline resistance of the heater (724) and a second predetermined operating index characterizing a resistance of the heater in a short condition wherein, optionally, the heater event comprises one of a short condition, an open circuit condition, or a failure condition wherein, optionally, the step of adjusting the power signal supplied to the heater comprises decreasing the power signal to the heater when the heater event is determined to correspond to a short condition.

FIG. 1

FIG. 2

300

306

302

304

**FIG. 3**

400

408 406 408 402 408

408 404 408 408

**FIG. 4**

**FIG. 5**

**FIG. 6**

Resistive data real time tracking and analysis for heating wire detection and monitoring

**FIG. 7**

EP 4 696 261 A1

**FIG. 8**

EP 4 696 261 A1

FIG. 9

1000

START

OBTAIN CRYOPROBE
OPERATING INFO. —— 1002

COMPARE
CRYOPROBE
OPERATING INFO. TO —— 1004
PREDETERMINED
OPERATING LEVEL(S)

1008

ADJUST
POWER
SIGNAL TO
HEATER

1006

HAS HEATER
EVENT
OCCURRED?

Y

N

1010

IS HEATER
PROCEDURE
COMPLETE?

N

Y

END

FIG. 10

**FIG. 11**

Europäisches
Patentamt
European
Patent Office
Office européen
des brevets

# EUROPEAN SEARCH REPORT

**Application Number**

EP 25 19 3160

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2023/178274 A1 (VARIAN MED SYS INC [US]) 21 September 2023 (2023-09-21) | 1-4,6-8, 15 | INV. A61B18/02 |
| Y | * paragraphs [0044], [0047], [0050], [0064], [0065], [0074] - [0076]; figures 1,2,5,7 * | 5,9-14 | |
| | ----- | | |
| X | WO 2023/177828 A1 (VARIAN MED SYS INC [US]) 21 September 2023 (2023-09-21) * paragraphs [0039], [0057] - [0061], [0084]; figures 1,4 * | 1-4,6-8, 15 | |
| | ----- | | |
| X | WO 2012/142448 A1 (GALIL MEDICAL INC [US]; DAVIS TIMOTHY J [US] ET AL.) 18 October 2012 (2012-10-18) * paragraphs [0022], [0023], [0029], [0054] - [0056]; figure 1A * | 1-4,6-8, 15 | |
| | ----- | | |
| Y | WO 2020/163854 A1 (UNIV EMORY [US]; FOCUSED CRYO INC [US]) 13 August 2020 (2020-08-13) * paragraphs [0084] - [0086]; figure 3A * | 5 | |
| | ----- | | **TECHNICAL FIELDS SEARCHED (IPC)** |
| Y | EP 2 415 416 A1 (CONMED CORP [US]) 8 February 2012 (2012-02-08) * paragraphs [0093] - [0099] * | 9-14 | A61B |
| | ----- | | |

The present search report has been drawn up for all claims

| Place of search | Date of completion of the search | Examiner |
|---|---|---|
| The Hague | 5 December 2025 | Mayer-Martenson, E |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

...................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 19 3160

This annex lists the patent family members relating to the patent documents cited in the above-mentioned European search report.
The members are as contained in the European Patent Office EDP file on
The European Patent Office is in no way liable for these particulars which are merely given for the purpose of information.

05-12-2025

| Patent document cited in search report | | Publication date | Patent family member(s) | | Publication date |
|---|---|---|---|---|---|
| WO 2023178274 | A1 | 21-09-2023 | CN | 118891013 A | 01-11-2024 |
| | | | EP | 4493088 A1 | 22-01-2025 |
| | | | US | 2023293218 A1 | 21-09-2023 |
| | | | WO | 2023178274 A1 | 21-09-2023 |
| WO 2023177828 | A1 | 21-09-2023 | CN | 118922140 A | 08-11-2024 |
| | | | EP | 4493086 A1 | 22-01-2025 |
| | | | US | 2023300949 A1 | 21-09-2023 |
| | | | WO | 2023177828 A1 | 21-09-2023 |
| WO 2012142448 | A1 | 18-10-2012 | US | 2012265189 A1 | 18-10-2012 |
| | | | WO | 2012142448 A1 | 18-10-2012 |
| WO 2020163854 | A1 | 13-08-2020 | EP | 3920784 A1 | 15-12-2021 |
| | | | US | 2022133381 A1 | 05-05-2022 |
| | | | WO | 2020163854 A1 | 13-08-2020 |
| EP 2415416 | A1 | 08-02-2012 | AU | 2011202872 A1 | 09-02-2012 |
| | | | CA | 2744462 A1 | 23-01-2012 |
| | | | EP | 2415416 A1 | 08-02-2012 |
| | | | JP | 5986717 B2 | 06-09-2016 |
| | | | JP | 2012024583 A | 09-02-2012 |
| | | | US | 2012022517 A1 | 26-01-2012 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 80076624